# EUROPEAN PATENT SPECIFICATION

(11) **EP 3 241 023 B1**
(45) Date of publication and mention of the grant of the patent: **18.03.2020**
(21) Application number: 15817318.7
(22) Date of filing: 21.12.2015
(51) Int. Cl.: G01N 33/00, G01N 25/18, G01N 27/62

(54) **METHOD AND SYSTEM FOR ASSESSING A PRODUCT**
VERFAHREN UND SYSTEM ZUR BEWERTUNG EINES PRODUKTS
PROCÉDÉ ET SYSTÈME POUR ÉVALUER UN PRODUIT

(30) Priority: 29.12.2014 EP 14200421
(43) Date of publication of application: 08.11.2017
(73) Proprietor: JT International SA, 1211 Geneva 26 (CH)
(72) Inventor: FRENTZEL, Vincent Bruno, 54296 Trier (DE); JANSER, Sven, 54570 Densborn (DE)
(74) Representative: Isarpatent
(86) International application number: PCT/EP2015/080808
(87) International publication number: WO 2016/107780

(56) References cited:
- GB-A- 2 324 870
- US-A1- 2004 090 235
- GILLES ADAM ET AL: "Evaluation of an electronic nose for the early detection of organic overload of anaerobic digesters", BIOPROCESS AND BIOSYSTEMS ENGINEERING, SPRINGER, BERLIN, DE, vol. 36, no. 1, 30 May 2012 (2012-05-30), pages 23-33, XP035156981, ISSN: 1615-7605, DOI: 10.1007/S00449-012-0757-6
- DUCERE J-M ET AL: "A computational chemist approach to gas sensors: Modeling the response of SnO2 to CO, O2, and H2O Gases", JOURNAL OF COMPUTATIONAL CHEMISTRY 20120130 JOHN WILEY AND SONS INC. USA, vol. 33, no. 3, 30 January 2012 (2012-01-30), pages 247-258, XP002742535, DOI: 10.1002/JCC.21959

## Description

The invention relates to a method and system for assessing a product, especially a smoking article, such as a cigarette or the like, or a smoking article filter element. More particularly, the invention relates to a method and system that is designed to inspect and/or to analyse such a product to assure product quality or integrity.

Smoking articles, such as cigarettes or the like, are popular consumer products that typically have a generally cylindrical rod-shaped configuration and include a charge, roll, or column of a smokable material, such as shredded tobacco (e.g. in cut filler form), surrounded by a paper wrapper forming a so-called "tobacco rod". A cigarette will also usually have a cylindrical filter element aligned in end-to-end relationship with the tobacco rod. The filter element may, for example, comprise cellulose acetate tow, and the tow is circumscribed by a paper material known as "plug wrap". Typically, the filter element is attached to one end of the tobacco rod using a circumscribing wrapping material known as "tipping paper".

In more recent years, various proposals have been made for modifying the sensory attributes of smoking articles, and particularly cigarettes, by using the filter elements as vehicles for adding flavour to mainstream smoke of the cigarettes. For example, these proposals have included introducing smoke-altering objects, such as breakable flavour pellets or capsules, flavoured or non-flavoured strands, exchange resin beads, adsorbent/absorbent particles, or combinations thereof, into cigarette filters in an automated fashion. During manufacture of the filter elements, the filter material is formed into a continuous filter rod having such objects positioned within that rod; for example, along a longitudinal axis thereof. The continuous filter rod is then divided or cut at predetermined intervals to form a plurality of filter elements, such that each filter element includes at least one of the objects therein.

The processes for inserting objects within a filter rod may produce some defective filter elements, however. For example, one or more of the objects inserted within a filter rod may be inadvertently omitted, may be incorrectly oriented, or, in the case of breakable pellets or capsules, may be inadvertently broken. Accordingly, it would be desirable to detect defective filter rods or defective filter elements such that they can be removed from the manufacturing process.
US2004/0090235 provides an example of a flavour monitoring system for smoking articles.

It is therefore an object of the present invention to provide an improved method and system for assessment of a product, especially of a smoking article or a filter element thereof. In particular, it is an object of the invention to increase the yield of the manufacturing process for this type of smoking article and/or to more reliably prevent smoking articles with a defective filter element from reaching a consumer. In accordance with this invention, a method and a system for assessing a product, especially a smoking article, according to the independent claims 1 and 7 respectively, is provided. Advantageous or preferred features of the invention are recited in the dependent claims.

According to one aspect, therefore, the invention provides a method of assessing a product, especially a smoking article or a filter element or filter rod for a smoking article, to determine product quality and/or product integrity, comprising the steps:
passing a gas sample through or around the product;
directing the gas sample to a sensor assembly downstream of the product for detecting at least one chemical compound, especially a flavour compound; and
analysing the gas sample with the sensor assembly to detect the at least one chemical compound in the gas sample.

In the event that a breakable pellet or capsule inserted into a filter rod or a filter element of a smoking article has broken or ruptured during manufacture of the smoking article, the chemical flavouring or aroma compound contained in pellet or capsule is able to leak out into the surrounding filter material. With the method of the invention, therefore, the presence of a broken pellet or capsule in a smoking article or filter element can be determined from the analysis of the gas sample. In particular, if the sensor assembly detects the particular flavour compound in the gas sample, especially at a predetermined or threshold level, this provides a reliable indication that the pellet or capsule in the filter element containing that flavour compound is broken. In particular, the flavour compound which leaks from the pellet or capsule is picked up by or transferred to the gas sample during the step of passing the gas sample through the product. The gas sample is obtained by passing a gas, such as compressed air, through the smoking article (e.g. cigarette) or through several smoking articles simultaneously. The gas sample is then analysed by the sensor assembly which provides either a positive or negative response. As noted above, the gas of the sample is preferably air, but other gases may also be contemplated.

In a preferred embodiment of the method, the step of passing the gas sample through the product, and especially through the smoking article or filter element, comprises: holding the product (e.g. the smoking article) in a testing zone between a gas inlet and a gas outlet; and generating a flow or stream of the gas sample through the testing zone from the inlet to the outlet. In this regard, the smoking article may be held with one end thereof at the gas inlet and the other end at the gas outlet, such that the flow of the gas sample passes longitudinally through the smoking article, i.e. through the filter element. The step of generating the flow or stream of the gas sample through the testing zone may include introducing a pressurized sample gas at the inlet of the testing zone. Alternatively, or in addition, the step of generating the flow or stream of the gas sample through the testing zone may comprise connecting the outlet of the testing zone to an under-pressure or vacuum source. The flow-rate of the gas sample can be controlled by controlling or varying the pressure difference across the product (e.g. across the smoking article) between the inlet and outlet. A combination of over-pressure and under-pressure has proved to yield the best results (high flow rate with no damage to the cigarette), but either over-pressure or under-pressure can be used alone.

In accordance with the invention, the step of passing the gas sample through the product comprises streaming the gas sample through the product at a flow rate in the range of about 0.1 l/min to about 20 l/min, and more preferably in the range of about 5 l/min to 15 l/min. The flow rate of the gas sample through the product(s) - i.e. the smoking article or cigarette - is important as it determines the travel time between the filter element (e.g. flavour capsule) and the sensor assembly. The sum of the travel time and measurement time should desirably enable the cigarette(s) that has/have been analysed to be rejected in the event that a defective product is detected. In a cigarette making machine, available time for testing and possible rejection is quite short and typically ranges from about 120ms to 300ms depending on the type of machine. Hence, the flow rate of the gas sample has an impact on the assessment method. In theory, higher gas sample flow rates equate to more dilute concentrations of volatiles in the gas stream and should have an impact on the detection speed. In practice, however, this has not been observed for the flow rates above. For the same reason, i.e. reducing the assessment time for high-speed applications, the implementation of the method should desirably minimize the travel distance for the gas sample from the product (e.g. the smoking article or filter element) to the sensor assembly.

In a preferred embodiment, the sensor assembly comprises at least one sensor selected from the group of: metal oxide sensors (MOS), thermo-conductivity sensors (TCS), and flame-ionization sensors (FIS). The sensor measurement time in the sensor assembly typically ranges from about 10ms to 100ms depending on the model and/or the manufacturer of the sensor(s). Although several types of gas sensors are applicable for this system, the preferred embodiments have focused on the use of metal oxide sensors (MOS), and especially tin oxide based sensors. This choice was driven by availability and cost considerations for these devices, as well as early experimental results. From a construction standpoint, these sensors include a heating circuit which degrades gas molecules entering a sensor chamber and a mesh-like structure (metal oxide lattice) which traps and oxidizes them. The resistance through the lattice determines the presence or absence of oxidized species. The sensor exhibits a different sensitivity/selectivity and recovery profile based on its operating temperature. Importantly, sudden changes in temperature, for example during start/stop operation where air flow is initiated or ceased, cause the sensor to reach a different zero point. From a dynamics perspective, these sensors exhibit an initial fast rise following exposure to the chemical compound with a maximum followed by a slow decrease back to a baseline level. From the structure description above, the reaction process is initially (forced) diffusion driven where the sample is in excess and flowing through the lattice, then reaction driven as the lattice catalytically oxidizes the sample which desorbs.

In a preferred embodiment, the method further comprises a step of heating the gas sample downstream of the product before the step of analysing the gas sample with the sensor assembly. In this context, metal oxide sensors generally rely on a heating element to degrade chemicals in the sample gas. Additionally, operating temperature has an impact on the resistance of the sensing circuit. Maintaining a constant and stable measurement temperature is thus relevant to the selectivity of the sensor and its accuracy. Under certain conditions, e.g. high sample flow-rate, maintaining the correct operating temperature might prove beyond the capacity of the built in heater of the sensor, causing it to lose sensitivity. For this reason, it can be useful to heat the gas sample, e.g. downstream of the product, before or during delivery of the gas sample to the sensor assembly for the analysing step.

In accordance with the invention, the sensor assembly comprises a plurality of sensors. The step of directing the gas sample to the sensor assembly comprises switching a flow path of the gas sample from one sensor to another sensor after detecting the chemical compound in the sensor assembly. Because the sensors, and especially metal oxide sensors, may require a recovery time or a regeneration time after detecting the compound before they can be effectively reused, this represents a limitation for high-speed applications such as in cigarette making machines. For example, a successful detection on a single cigarette could prevent a correct determination on the following ten as the sensor needs time to recover from the first exposure. For this reason, it is contemplated that the sensor assembly includes a plurality of sensors arranged in parallel such that it is possible to switch to a fresh or a regenerated sensor after a detection in the sensor assembly. In this scheme, one sensor may be in operation while the others (with their exact number depending on the expected positive detection count) are maintained in a standby mode. Upon a detection, the flow of the next gas sample is switched from the current sensor to one of the others in standby mode and the previous sensor then undergoes regeneration before it is placed on standby mode again.

In a particularly preferred embodiment, therefore, the method further comprises a step of regenerating a sensor of the sensor assembly after detecting the at least one chemical compound in the gas sample. The step of regenerating the sensor preferably comprises passing a clean gas flow to that sensor. The clean gas flow may, for example, comprise a flow of clean air and/or a flow of oxygen to promote a faster regeneration or recovery of the sensor. Further, the step of regenerating the sensor preferably comprises heating the clean gas flow to that sensor. In this regard, heating of the clean gas flow may accelerate regeneration or recovery of the metal oxide sensor (MOS) by increasing the degradation rate of the adsorbed volatiles deposited on its lattice.

In a preferred embodiment, the step of analysing the gas sample with the sensor assembly comprises establishing a response signal from the sensor assembly and comparing the response signal with a reference signal to detect a presence of the at least one chemical compound in the gas sample. To this end, the detection may typically be achieved via either or a combination of: comparison of a current value from the sensor with a predetermined threshold, whereby crossing the threshold triggers a response; and/or by inspection of a rate of change of the value given a certain historical range, and comparison with a predetermined threshold. For high speed applications, the latter alternative will be preferred with added processing to account for the sensor recovery time.

In a preferred embodiment, the method further comprises a step of diluting the gas flow downstream of the product before the step of analysing the gas flow with the sensor assembly. In this regard, the volatile concentration (i.e. the concentration of the chemical or flavor compound) in the stream of sample gas will typically have an impact on the speed of detection, on the amplitude of the response, and on the subsequent recovery profile of the sensor. For this reason, the gas sample may be diluted, e.g. via a valve to introduce a diluting gas, to decrease the concentration of the volatile (i.e. of the chemical or flavor compound) and thereby speed up the sensor recovery.

In a preferred embodiment, the method comprises the step of rejecting a product, e.g. a smoking article, which is detected as being defective in the analysing step. This preferably comprises ejecting or removing the defective smoking article from a transport path of the cigarette making machine, for example, by imparting an impulse to the defective smoking article.

In a preferred embodiment of the invention, the products or smoking articles are provided or arranged in the testing zone for assessment individually or separately from one another. In an alternative preferred embodiment, however, a plurality of the products or smoking articles can be provided or arranged in the testing zone for assessment collectively or in groups. In such a case, in the event of a positive detection of a defective product or smoking article, it may be pragmatic to reject the entire group of products or smoking articles rather than then further test each one individually.

In a preferred form, the method of the invention is carried out after manufacture or assembly of the smoking articles and before packaging. By arranging the testing zone adjacent to and/or immediately upstream of packaging equipment, the yield of the manufacturing process and the reliability of the smoking articles overall can be improved. In this regard, a position immediately prior to the packaging of the smoking articles provides a final opportunity to sort defective smoking articles from the packaging equipment, and thereby to prevent them from inclusion in the final packaged product to be dispatched to consumers. The testing zone may be in a transport path provided to convey the smoking articles from the manufacturing equipment to the packaging equipment for packaging the smoking articles into packs. In this regard, the testing zone may be associated with or incorporated in a buffer for temporarily storing an accumulation of the smoking articles adjacent to, or immediately upstream of, the packaging equipment.

According to another aspect, the invention provides a system for assessing a product, especially a smoking article or filter element, to determine product quality and/or product integrity, comprising:
holder means for holding the product in a testing zone;
means for passing a gas sample through the testing zone; and
a sensor assembly arranged downstream of the testing zone to which the gas sample is directed for detecting a chemical compound, especially a flavour or aroma compound, in the gas sample.

As noted above, in accordance with the invention the product is held in the testing zone by the holder means between a gas inlet and a gas outlet. Thus, the product (e.g. smoking article) may be held with one end thereof at the gas inlet and the other end at the gas outlet, such that the gas sample passes longitudinally through the smoking article, i.e. through the filter element. To this end, the holder means may comprise a first holder member at the inlet of the testing zone and a second holder member at the outlet of the testing zone. The first and second holder members preferably include one or more sealing elements for engaging and sealing around the product.

In accordance with the invention, the means for passing the gas sample through the testing zone comprises means for generating a flow or stream of the gas sample through the testing zone from the inlet to the outlet. The means for generating the flow or stream of a gas sample through the testing zone may, for example, include a pressurized sample gas, such as pressurized air, connected at the inlet of the testing zone. Alternatively, or in addition, the means for generating the flow or stream of the gas sample through the testing zone may comprise a source of an under-pressure or suction connected to the outlet of the testing zone.

In a preferred embodiment, the sensor assembly of the system comprises at least one sensor and a signal processing device configured to establish a response signal from the sensor and to compare the response signal with a reference signal to detect the presence of the chemical compound in the gas sample from an outlet of the testing zone. The at least one sensor of the sensor assembly is preferably selected from the group consisting of: metal oxide sensors (e.g. tin oxide sensors), thermo-conductivity sensors, and flame-ionization sensors.

In accordance with the invention, the sensor assembly comprises a plurality of sensors which are arranged such that it is possible to direct the gas sample from the testing zone to any one of the sensors. That is, the system preferably comprises a switching mechanism for switching flow of the gas sample between the plurality of sensors. The switching mechanism may comprise an arrangement of conduits for conveying the gas sample to each sensor and valves for controlling the flow of the gas sample through the respective conduits. Further, the switching mechanism may comprise a controller, typically including a computer processor, for controlling operation of the valves for directing the gas sample to a respective sensor of the sensor assembly. As discussed above, one sensor of the sensor assembly may be in operation while the others (with their exact number depending on the expected positive detection count) are maintained in a standby mode. Upon detection of a flavour compound with one sensor, the flow of the next gas sample is then switched from the current sensor to another one of the sensors in standby mode. The previous sensor then undergoes regeneration before it enters standby mode again.

In a preferred embodiment, the system further includes means for diluting the gas sample downstream of the testing zone and upstream of the sensor assembly. In this regard, it has been noted that diluting or decreasing the concentration of the chemical or flavour compound in the gas sample can speed up the recovery of the sensor(s) in the sensor assembly. To this end, the diluting means may include a conduit and/or valve configuration for introducing a diluting gas, such as air, into the gas sample after the testing zone and upstream of the sensor assembly. The diluting means may also comprise a controller, e.g. having a computer processor, for controlling operation of the valve(s)

In a preferred embodiment, the system further comprises at least one heater for heating the gas sample downstream of the testing zone but before it reaches the sensor assembly. In this context, it has been noted that the operating temperature of the sensor assembly, and especially of a metal oxide sensor, has an impact on the resistance of the sensing circuit, and that maintaining a constant and stable measurement temperature is thus important to the selectivity of the sensor and its accuracy. Under certain conditions, such as high gas sample flow-rate, therefore, a heater is useful for maintaining a desired operating temperature, which might otherwise be beyond the capacity of the built-in heating device of the sensor itself, causing it to lose sensitivity.

In a preferred embodiment, the system includes an arrangement for regenerating each sensor in the sensor assembly after a positive detection. The regenerating arrangement preferably includes means for supplying or conveying a regenerator gas or a "clean" gas (i.e. free of the chemical compound to be detected) to each sensor in the sensor assembly. For example, the regenerator gas or "clean" gas may comprise any one or more of air, oxygen, and/or nitrogen. Furthermore, the regenerating arrangement may comprise at least one heater configured for heating the regenerator gas or "clean" supplied or conveyed to each sensor in the sensor assembly. In this regard, the recovery or regeneration of a metal oxide sensor can be accelerated by increasing the degradation rate of the adsorbed volatiles deposited on its lattice, a process that can be sped up with increased temperature.

In a particularly preferred embodiment, the system of the invention may include a sorting means for removing individual smoking articles from a transport path to a packaging assembly if they are detected by the at least one sensor as defective. In this regard, the sorting means may be designed to eject or remove a defective smoking article from the transport path, for example, by imparting an impulse to the defective smoking article.

For a more complete understanding of the invention and the advantages thereof, exemplary embodiments of the invention are explained in more detail in the following description with reference to the accompanying drawing figures, in which like reference characters designate like parts and in which:
- Fig. 1: is a schematic illustration of a method and system for assessing a smoking article according to a basic embodiment of the invention;
- Fig. 2: is a schematic side view of a system according to the embodiment of Fig. 1;
- Fig. 3: is a graph schematically illustrating a response curve of a sensor in a method and system for assessing a smoking article according to an embodiment of the invention; and
- Fig. 4: is a schematic illustration of a method and system for assessing a smoking article according to another embodiment of the invention.

The accompanying drawings are included to provide a further understanding of the present invention and are incorporated in and constitute a part of this specification. The drawings illustrate particular embodiments of the invention and together with the description serve to explain the principles of the invention. Other embodiments of the invention and many of the attendant advantages of the invention will be readily appreciated as they become better understood with reference to the following detailed description.

It will be appreciated that common and well understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

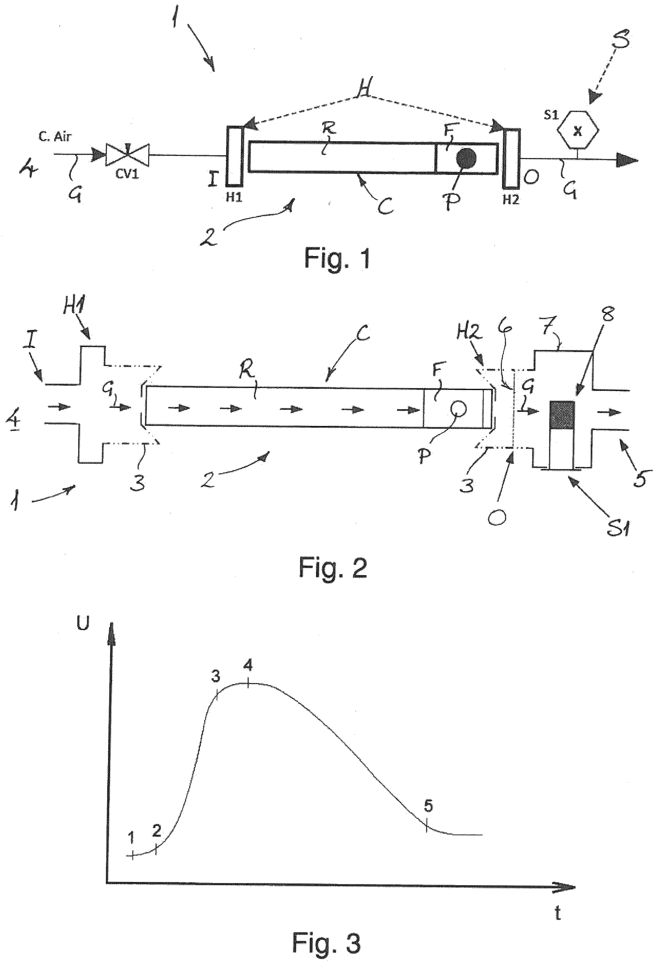

Referring firstly to Figs. 1 and 2 of the drawings, a system 1 for assessment of smoking articles, such as cigarettes, according to a simple embodiment is shown schematically. The system 1 may desirably be incorporated in a facility or machine for producing and/or packaging cigarettes. A single smoking article or cigarette C is shown having a generally cylindrical rod-shaped configuration and including a tobacco rod R of cut tobacco wrapped in cigarette paper, and a filter element F aligned in end-to-end relationship and connected to the tobacco rod R via tipping paper. The filter element F can be seen to include a breakable capsule or pellet P embedded therein which contains a liquid flavouring compound.

As seen in drawing Figs. 1 and 2, the system 1 includes a holder mechanism H for holding the cigarette C in a testing zone 2 between a gas inlet I and a gas outlet O. In this regard, the holder mechanism H comprises a first holder member H1 for holding one end of the cigarette C at the inlet I of the testing zone 2 and a second holder member H2 for holding the other end of the cigarette C at the outlet O of the testing zone 2. The first and second holder members H1, H2 include relatively soft and flexible sealing elements 3 for engaging and sealing around the ends of the cigarette C.

The system 1 furthermore includes means for generating a flow or stream of a gas sample G (in this case, air) through the testing zone 2 from the inlet I to the outlet O, such that the gas sample G passes longitudinally through the cigarette C, i.e. through the filter element F. Specifically, this embodiment of the system 1 includes a supply 4 of pressurized air as the sample gas G connected via a line or conduit in communication with the gas inlet I to the testing zone 2 to generate the gas flow. A suction source 5 may also be connected in communication with the gas outlet O from the testing zone 2 to promote the flow of the gas sample. The flow of the gas sample G from the supply 4 through the testing zone 2 is regulated or controlled by a control valve CV1 and the flow rate typically lies within the range of about 5 l/min to about 15 l/min. The control valve CV1 may, for example, be a needle valve and may be used to regulate (e.g. reduce) the pressure of the gas.

In addition, the system 1 for assessing the smoking article or cigarette C includes a sensor assembly S arranged in a flow path of the gas sample G downstream of the testing zone 2. In this embodiment, the sensor assembly S comprises a single gas sensor S1 of the metal oxide (MOS) type, especially a tin oxide sensor, which are readily available, have a relatively low cost, and provide satisfactory results, and a signal processing device (not shown) configured to establish a response signal from the sensor S1 and to compare the response signal with a reference signal for detecting the presence of the flavour compound X in the gas sample G. Thus, after passing through the cigarette C, the gas sample G is directed to the sensor assembly S (e.g. via a flow conditioner element 6) for detecting a particular flavour compound X in the gas sample G. The metal oxide sensor S1 includes a heating circuit which degrades gas molecules entering the sensor chamber or housing 7 and a mesh-like structure 8 (i.e. a metal oxide lattice) which traps and oxidizes them. The resistance through the lattice 8 determines the presence or absence of oxidized species.

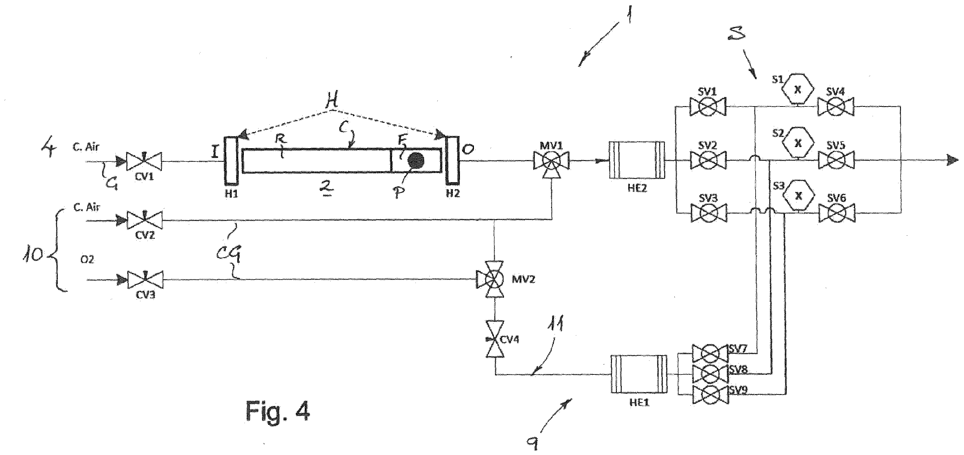

With reference now also to Fig. 3 of the drawings, the sensor S1 exhibits different sensitivity/selectivity and recovery profile based on its operating temperature. At point (1) in Fig. 3, a gas sample is introduced and stopped after i.e. 5 ms. At point (2) in Fig. 3, there is an initial sign of a detection of the flavour compound X. The sensor S1 exhibits an initial fast rise in the period from point (2) to point (3) following exposure to the gas sample G carrying the flavour compound X with a maximum value reached at point (4). This is followed by a slow decrease or recovery back to a baseline in the period from point (4) to point (5). The reaction process is initially (forced) diffusion driven where the gas sample G is in excess and flowing through the lattice 8 and then reaction driven as the lattice catalytically oxidizes the flavour compound X which desorbs. It will be noted that during the initial fast rise - i.e. in the period from point (2) to point (3) - interrupting the flow of the gas sample G does not prevent the sensor S1 from reaching its maximum value at point (4). Accordingly, the sensor S1 is essentially ineffective or "blind" until after the recovery or regeneration period to point (5), which in turn creates a limitation for high-speed applications. This limitation is addressed in the preferred embodiment of Fig. 4 described below. In view of this characteristic curve for the reaction or behaviour of the metal oxide sensor S1, detection of the flavour compound X will be achieved via either one or a combination of: (i) comparing the sensor current with a predetermined threshold value, such that crossing the threshold triggers a response; or (ii) inspecting the rate of change of the current given a certain historical range, and comparing with a threshold. The second alternative is preferred for high speed applications, with added processing to account for the sensor recovery time.

Referring now to Fig. 4 of the drawings, a system 1 for assessing smoking articles, such as cigarettes, according to a more sophisticated embodiment of the invention is shown schematically. This embodiment addresses the limitation of the recovery or regeneration time required by an individual sensor of the sensor assembly S by providing the sensor assembly S with a plurality of sensors S1, S2, S3 arranged in parallel and connected for selective communication with the outlet O of the testing zone 2 via respective valves, such as solenoid valves SV1, SV2, SV3. In this way, a single one of the sensors S1, S2, S3 will be in operation at any given time (e.g. first sensor S1) while the remaining sensors (e.g. second and third sensors S2, S3) are in a standby mode. In the event that the first sensor S1 detects the flavour compound X in the gas sample G, a controller (not shown) that includes the signal processing device (not shown) of the sensor assembly S, controls operation of the valves SV1, SV2, SV3 to isolate the first sensor S1 (i.e. by closing first solenoid valve SV1) while that sensor undergoes a recovery or regeneration procedure and to direct the next gas sample G to one of the other sensors in standby mode, e.g. the second sensor S2 by opening second solenoid valve SV2. The third sensor S3 thus remains on standby (i.e. with third valve SV3 closed) while the second sensor S2 is in use and the first sensor S1 undergoes recovery or regeneration.

In this embodiment, the recovery or regeneration of each sensor S1, S2, S3 may be promoted or accelerated by a regeneration arrangement 9, which is configured to direct a clean gas CG, such as compressed air and/or oxygen, not containing any flavour compound X from an extra gas supply 10 to the respective sensor in a recovery or regeneration procedure. The supply 10 of the clean gas CG includes a separate compressed air supply and an oxygen supply, each of which is controlled or regulated via a respective control valve CV2, CV3. The clean gas CG, i.e. of air and/or oxygen, is delivered or conveyed to the sensor S1, S2, S3 via a pathway or conduit 11 that bypasses the testing zone 2 and permits mixture of these gases via a mixing valve MV2. A further control valve CV4 in the regenerator pathway or conduit 11 may regulate the pressure of the clean gas mixture delivered to the respective sensor. The regeneration arrangement 9 further includes a heater HE1 for pre-heating the clean gas CG before it is delivered or fed to the sensor to be regenerated. In this regard, a higher temperature of the clean gas G can increase the degradation rate of the adsorbed volatiles deposited on the lattice of a metal oxide sensor, which in turn can accelerate the recovery of the respective sensor S1, S2, S3. Also, oxygen added to the clean gas CG recovery stream is deemed to support the oxidation reaction, which is essentially first order with respect to O₂. Each of three solenoid valves SV7, SV8, SV9 is controlled via the controller (not shown) to selective direct the recovery gas stream CG on the pathway 11 from the heater HE1 to the particular sensor (only) undergoing recovery or regeneration.

Meanwhile, the next cigarette C to be tested has already been introduced to or placed in the testing zone 2 and the next assessment has been carried out. For example, a cigarette C which had tested positive for a broken or ruptured capsule or pellet P by sensor S1 was removed from the holders H in the testing zone 2 and ejected to a waste collection to prevent it from transport to packaging equipment and the next cigarette C to be tested was then conveyed or delivered to the testing zone 2, where it was held by the holder mechanism H as the next gas sample G of compressed air from the supply 4 was directed through that cigarette C in testing zone 2 and further conveyed or directed to the second sensor S2 of the sensor assembly S for analysis while the first sensor is regenerated.

With reference again to a path of the gas sample G from the testing zone 2 to the sensor assembly S, it will be noted that the extra gas supply 10 also enables clean gas G comprising either or both of compressed air and/or oxygen to be introduced into gas sample G downstream of the testing zone 2 and upstream of the sensor assembly S via a mixing valve MV1 (in combination with the valves CV2, CV3 and MV2). This operates to dilute a concentration of any flavour compound X in the gas sample G before analysis by the sensor assembly, as the concentration of the volatile in the gas sample stream impacts on the speed of detection, amplitude of the response and the subsequent recovery profile of the sensors S1, S2, S3. By diluting the gas sample to decrease the concentration, therefore, it is possible to increase the speed of sensor recovery. Furthermore, a second heater device HE2 is included in the path of the gas sample G between the testing zone 2 and the sensor assembly S because maintaining a stable measurement temperature is important to the selectivity and accuracy of metal oxide sensors. By heating the gas sample G to a predetermined temperature upstream of sensor assembly S, an internal heating element in each metal oxide sensors S1, S2, S3 can be assisted to help ensure a correct operating temperature.

Although specific embodiments of the invention have been illustrated and described herein, it will be appreciated by those of ordinary skill in the art that a variety of alternate and/or equivalent implementations exist. It should be appreciated that the exemplary embodiment or exemplary embodiments are only examples, and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents. Generally, this application is intended to cover any adaptations or variations of the specific embodiments discussed herein.

In this document, the terms "comprise", "comprising", "include", "including", "contain", "containing", "have", "having", and any variations thereof, are intended to be understood in an inclusive (i.e. non-exclusive) sense, such that the process, method, device, apparatus or system described herein is not limited to those features or parts or elements or steps recited but may include other elements, features, parts or steps not expressly listed or inherent to such process, method, article, or apparatus. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

### List of Reference Signs

- 1: system
- 2: testing zone
- 3: sealing element
- 4: gas supply
- 5: suction source
- 6: flow conditioner element
- 7: sensor chamber or housing
- 8: metal lattice or mesh-like structure
- 9: regeneration arrangement
- 10: extra gas supply
- 11: regenerator pathway
- R: tobacco rod
- F: filter element
- P: capsule or pellet
- C: cigarette
- G: gas sample
- H: holder mechanism
- H1: first holder member
- H2: second holder member
- CV1: control valve
- CV2: control valve
- CV3: control valve
- CV4: control valve
- I: gas inlet
- O: gas outlet
- S: sensor assembly
- S1: first sensor
- S2: second sensor
- S3: third sensor
- SV1: first solenoid valve
- SV2: second solenoid valve
- SV3: third solenoid valve
- SV4: fourth solenoid valve
- SV5: fifth solenoid valve
- SV6: sixth solenoid valve
- SV7: seventh solenoid valve
- SV8: eighth solenoid valve
- SV9: ninth solenoid valve
- MV1: mixing valve
- MV2: mixing valve
- HE1: heater device
- HE2: heater device

## Claims

1. A method for assessing a product (C), especially a smoking article or a filter element therefor, to determine product quality and/or product integrity, the method comprising the steps of:
passing a gas sample (G) through the product (C);
directing the gas sample (G) to a sensor assembly (S) downstream of the product (C) for detecting at least one flavour compound (X); and
analysing the gas sample (G) with the sensor assembly (S) to detect the at least one flavour compound (X) in the gas sample (G);
**characterised in that**
- holding the product (C) or smoking article in a testing zone (2) between a gas inlet (I) and a gas outlet (O) and generating a flow or stream of the gas sample (G) through the testing zone (2) from the gas inlet (I) to the gas outlet (O), wherein the testing zone (2) is located in a transport path which is provided to convey the product from a manufacturing equipment to a packaging equipment; and
- the step of passing the gas sample (G) through the product (C) comprises streaming the gas sample (G) through the product (C) at a flow rate in the range of about 0.1 l/min to about 20 l/min, and wherein the sensor assembly (S) comprises a plurality of sensors (S1, S2, S3) arranged in parallel, and wherein the step of directing the gas sample (G) to the sensor assembly (S) comprises switching a flow path of the gas flow (G) from one sensor (S1, S2, S3) to another sensor (S1, S2, S3) after detecting the flavour compound (X) in the gas sample (G).

2. A method according to claim 1, wherein the step of analysing the gas sample (G) with the sensor assembly (S) includes establishing a response signal from the sensor assembly (S) and comparing the response signal with a reference signal to detect a presence of the at least one flavour component (X) in the gas sample (G).

3. A method according to claim 1, wherein the step of generating a flow or stream of the gas sample (G) through the testing zone (2) comprises:
introducing pressurized gas at the gas inlet (I) of the testing zone (2); and/or
connecting the gas outlet (O) of the testing zone (2) to an under-pressure or vacuum source.

4. A method according to any one of the preceding claims, further comprising a step of:
diluting the gas sample (G) downstream of the product (C) before the step of analysing the gas sample (G) with the sensor assembly (S); and/or
heating the gas sample (G) downstream of the product (C) before the step of analysing the gas sample (G) with the sensor assembly (S).

5. A method according to any one of the preceding claims, further comprising a step of regenerating a sensor (S1, S2, S3) of the sensor assembly (S) after detecting the at least one flavour compound (X) in the gas sample (G); wherein the step of regenerating the sensor (S1, S2, S3) preferably includes passing a clean gas (CG) to that sensor.

6. A method according to any one of the preceding claims, wherein the sensor assembly (S) comprises at least one sensor (S1, S2, S3) selected from the group of: metal oxide sensors, thermo-conductivity sensors, and flame-ionization sensors.

7. A system (1) for assessing a product (C), especially a smoking article or a smoking article filter element, to determine product quality and/or product integrity, comprising:
holder means (H) for holding the product (C) in a testing zone (2) having a gas inlet (I) and a gas outlet (O);
means for providing or generating flow of a gas sample (G) through the testing zone (2) from the inlet (I) to the outlet (O); and
a sensor assembly (S) arranged downstream of the testing zone (2) to which the gas sample (G) is directed for detecting a flavour compound (X) in the gas sample (G),
**characterised in that**
- the testing zone (2) is located in a transport path which is provided to convey the product from a manufacturing equipment to a packaging equipment; and
- the system (1) further comprises a control valve (CV1) to regulate or control the flow of the gas sample (G) at a flow rate in the range of about 0.1 l/min to about 20 l/min, and wherein the sensor assembly (S) comprises a plurality of sensors (S1, S2, S3) arranged in parallel, and wherein for a step of directing the gas sample (G) to the sensor assembly (S) a flow path of the gas flow (G) is switchable from one sensor (S1, S2, S3) to another sensor (S1, S2, S3) after the flavour compound (X) in the gas sample (G) is detected.

8. A system (1) according to claim 7, wherein the sensor assembly (S) comprises at least one sensor (S1, S2, S3) and a signal processing device configured to establish a response signal from the sensor (S1, S2, S3) and to compare the response signal with a reference signal for detecting the presence of the flavour compound in the gas sample (G) from the outlet (O) of testing zone (6).

9. A system (1) according to claim 7 or claim 8, wherein the sensor assembly (S) comprises at least one sensor (S1, S2, S3) selected from the group consisting of: metal oxide sensors, such as tin oxide sensors, thermo-conductivity sensors, and flame-ionization sensors.

10. A system (1) according to any one of claims 7 to 9, wherein the means for generating the flow of the gas sample (G) through the testing zone (2) comprises a pressurized sample gas connected for introduction at the gas inlet (I) of the testing zone (2) and/or an under-pressure or a suction applied at the gas outlet (O) of the testing zone (2).

11. A system (1) according to any one of claims 7 to 10, wherein the holder means (H) for holding the product (C) comprises a first holder member (H1) at the inlet (I) of the testing zone (2) and a second holder member (H2) at the outlet (O) of the testing zone (2), wherein the first and second holder members (H1, H2) preferably include one or more sealing elements (4) for engaging and sealing around the product (C).

12. A system (1) according to any one of claims 7 to 11, further comprising:
means for diluting the gas sample (G) downstream of the testing zone (2) and upstream of the sensor assembly (S); and/or
at least one heater (HE) for heating the gas sample (G) downstream of the testing zone (2) and upstream of the sensor assembly (S).

13. A system (1) according to any of claims 7 to 12, further comprising means for regenerating each sensor (S1, S2, S3) in the sensor assembly (S), the regenerating means preferably comprising means for supplying air, oxygen and/or nitrogen to each sensor (S1, S2, S3).

## Patentansprüche

1. Verfahren zum Bewerten eines Produktes (C), insbesondere eines Rauchartikels oder eines Filterelements dafür, um die Produktqualität und/oder die Produktintegrität zu bestimmen, wobei das Verfahren die folgenden Schritte umfasst:
Leiten einer Gasprobe (G) durch das Produkt (C);
Leiten der Gasprobe (G) zu einer Sensoranordnung (S) stromabwärts des Produkts (C) zum Detektieren mindestens einer Aromaverbindung (X); und
Analyse der Gasprobe (G) mit der Sensoranordnung (S) zum Detektieren der mindestens einen Aromaverbindung (X) in der Gasprobe (G);
**dadurch gekennzeichnet, dass**
- das Produkt (C) oder der Rauchartikel in einer Testzone (2) zwischen einem Gaseinlass (I) und einem Gasauslass (O) gehalten wird und eine Strömung oder ein Strom der Gasprobe (G) generiert wird, der durch die Testzone (2) von dem Gaseinlass (I) zu dem Gasauslass (O) strömt,
wobei sich die Testzone (2) in einem Transportpfad befindet, der dafür vorgesehen ist, das Produkt von einer Herstellungsanlage zu einer Verpackungsanlage zu befördern; und
- der Schritt des Leitens der Gasprobe (G) durch das Produkt (C) umfasst, die Gasprobe (G) durch das Produkt (C) mit einer Strömungsrate im Bereich von etwa 0,1 l/min bis etwa 20 l/min strömen zu lassen, und wobei die Sensoranordnung (S) mehrere parallel angeordnete Sensoren (S1, S2, S3) umfasst, und wobei der Schritt des Leitens der Gasprobe (G) zu der Sensoranordnung (S) umfasst, einen Strömungspfad des Gasstroms (G) von einem Sensor (S1, S2, S3) zu einem anderen Sensor (S1, S2, S3) umzuschalten, nachdem die Aromaverbindung (X) in der Gasprobe (G) detektiert wurde.

2. Verfahren nach Anspruch 1, wobei der Schritt des Analysierens der Gasprobe (G) mit der Sensoranordnung (S) umfasst, ein Antwortsignal von der Sensoranordnung (S) zu erzeugen und das Antwortsignal mit einem Referenzsignal zu vergleichen, um das Vorhandensein der mindestens einen Aromakomponente (X) in der Gasprobe (G) zu detektieren.

3. Verfahren nach Anspruch 1, wobei der Schritt des Generierens einer Strömung oder eines Stromes der Gasprobe (G) durch die Testzone (2) Folgendes umfasst:
Einleiten von unter Druck stehendem Gas an dem Gaseinlass (I) der Testzone (2); und/oder
Verbinden des Gasauslasses (O) der Testzone (2) mit einer Unterdruck- oder Vakuumquelle.

4. Verfahren nach einem der vorangehenden Ansprüche, des Weiteren umfassend den folgenden Schritt:
Verdünnen der Gasprobe (G) stromabwärts des Produkts (C) vor dem Schritt des Analysierens der Gasprobe (G) mit der Sensoranordnung (S); und/oder
Erwärmen der Gasprobe (G) stromabwärts des Produkts (C) vor dem Schritt des Analysierens der Gasprobe (G) mit der Sensoranordnung (S).

5. Verfahren nach einem der vorangehenden Ansprüche, des Weiteren umfassend einen Schritt, einen Sensor (S1, S2, S3) der Sensoranordnung (S) zu regenerieren, nachdem die mindestens eine Aromaverbindung (X) in der Gasprobe (G) detektiert wurde;
wobei der Schritt des Regenerierens des Sensors (S1, S2, S3) bevorzugt umfasst, ein sauberes Gas (CG) zu diesem Sensor zu leiten.

6. Verfahren nach einem der vorangehenden Ansprüche, wobei die Sensoranordnung (S) mindestens einen Sensor (S1, S2, S3) umfasst, der ausgewählt ist aus der Gruppe bestehend aus Metalloxidsensoren, Wärmeleitfähigkeitssensoren und Flammenionisationssensoren.

7. System (1) zum Bewerten eines Produktes (C), insbesondere eines Rauchartikels oder eines Filterelements dafür, um die Produktqualität und/oder die Produktintegrität zu bestimmen, und umfassend:
ein Haltermittel (H) zum Halten des Produkts (C) in einer Testzone (2), die einen Gaseinlass (I) und einem Gasauslass (O) aufweist;
ein Mittel zum Bereitstellen oder Erzeugen einer Strömung einer Gasprobe (G) durch die Testzone (2) von dem Einlass (I) zu dem Auslass (O); und
eine Sensoranordnung (S), die stromabwärts der Testzone (2) angeordnet ist, zu der die Gasprobe (G) geleitet wird, um eine Aromaverbindung (X) in der Gasprobe (G) zu detektieren,
**dadurch gekennzeichnet, dass**
- sich die Testzone (2) in einem Transportpfad befindet, der dafür vorgesehen ist, das Produkt von einer Herstellungsanlage zu einer Verpackungsanlage zu befördern; und
- das System (1) des Weiteren ein Steuerventil (CV1) umfasst, um die Strömung der Gasprobe (G) auf eine Strömungsrate im Bereich von etwa 0,1 l/min bis etwa 20 l/min zu regeln oder zu steuern, und wobei die Sensoranordnung (S) mehrere parallel angeordnete Sensoren (S1, S2, S3) umfasst, und wobei für einen Schritt des Leitens der Gasprobe (G) zu der Sensoranordnung (S) ein Strömungspfad des Gasstroms (G) von einem Sensor (S1, S2, S3) zu einem anderen Sensor (S1, S2, S3) umgeschaltet werden kann, nachdem die Aromaverbindung (X) in der Gasprobe (G) detektiert wurde.

8. System (1) nach Anspruch 7, wobei die Sensoranordnung (S) mindestens einen Sensor (S1, S2, S3) und eine Signalverarbeitungsvorrichtung umfasst, die dafür eingerichtet ist, ein Antwortsignal von dem Sensor (S1, S2, S3) zu erzeugen und das Antwortsignal mit einem Referenzsignal zu vergleichen, um das Vorhandensein der Aromaverbindung in der Gasprobe (G) von dem Auslass (O) der Testzone (6) zu detektieren.

9. System (1) nach Anspruch 7 oder Anspruch 8, wobei die Sensoranordnung (S) mindestens einen Sensor (S1, S2, S3) umfasst, der ausgewählt ist aus der Gruppe bestehend aus Metalloxidsensoren, wie zum Beispiel Zinnoxidsensoren, Wärmeleitfähigkeitssensoren und Flammenionisationssensoren.

10. System (1) nach einem der Ansprüche 7 bis 9, wobei das Mittel zum Generieren der Strömung der Gasprobe (G) durch die Testzone (2) ein unter Druck stehendes Probengas umfasst, das zum Einleiten am Gaseinlass (I) der Testzone (2) angeschlossen ist, und/oder einen Unterdruck oder einen Sog umfasst, der am Gasauslass (O) der Testzone (2) angelegt wird.

11. System (1) nach einem der Ansprüche 7 bis 10, wobei das Haltermittel (H) zum Halten des Produkts (C) ein erstes Halterelement (H1) am Einlass (I) der Testzone (2) und ein zweites Halterelement (H2) am Auslass (O) der Testzone (2) umfasst, wobei das erste und das zweite Halterelement (H1, H2) bevorzugt ein oder mehrere Dichtungselemente (4) zum Ineingriffnehmen und Abdichten um das Produkt (C) herum umfassen.

12. System (1) nach einem der Ansprüche 7 bis 11, des Weiteren umfassend:
ein Mittel zum Verdünnen der Gasprobe (G) stromabwärts der Testzone (2) und stromaufwärts der Sensoranordnung (S); und/oder
mindestens eine Heizung (HE) zum Erwärmen der Gasprobe (G) stromabwärts der Testzone (2) und stromaufwärts der Sensoranordnung (S).

13. System (1) nach einem der Ansprüche 7 bis 12, des Weiteren umfassend ein Mittel zum Regenerieren jedes Sensors (S1, S2, S3) in der Sensoranordnung (S), wobei das Regenerierungsmittel bevorzugt ein Mittel zum Zuführen von Luft, Sauerstoff und/oder Stickstoff zu jedem Sensor (S1, S2, S3) umfasst.

## Revendications

1. Procédé pour évaluer un produit (C), en particulier un article à fumer ou un élément filtrant de celui-ci, afin de déterminer la qualité du produit et/ou l'intégrité du produit, le procédé comprenant les étapes consistant à :
faire passer un échantillon de gaz (G) à travers le produit (C) ;
diriger l'échantillon de gaz (G) vers un ensemble capteur (S) en aval du produit (C) pour détecter au moins un composé aromatique (X); et
analyser l'échantillon de gaz (G) avec l'ensemble capteur (S) pour détecter le au moins un composé aromatique (X) dans l'échantillon de gaz (G) ;
**caractérisé en ce que**
- le produit (C) ou l'article à fumer est maintenu dans une zone de test (2) entre une entrée de gaz (I) et une sortie de gaz (O) et un flux ou un écoulement de l'échantillon de gaz (G) à travers la zone de test (2) depuis l'entrée de gaz (I) vers la sortie de gaz (0) est généré,
dans lequel la zone de test (2) est située dans un trajet de transport qui est prévu pour acheminer le produit à partir d'un équipement de fabrication vers un équipement de conditionnement; et **en ce que**
- l'étape consistant à faire passer l'échantillon de gaz (G) à travers le produit (C) comprend de diffuser en continu l'échantillon de gaz (G) à travers le produit (C) à un débit dans la plage d'environ 0,1 l/min à environ 20 l/min, et dans lequel l'ensemble capteur (S) comprend une pluralité de capteurs (S1, S2, S3) disposés en parallèle, et dans lequel l'étape consistant à diriger l'échantillon de gaz (G) vers l'ensemble capteur (S) comprend de commuter un trajet d'écoulement du flux de gaz (G) à partir d'un capteur (S1, S2, S3) vers un autre capteur (S1, S2, S3), après la détection du composé aromatique (X) dans l'échantillon de gaz (G).

2. Procédé selon la revendication 1, dans lequel l'étape consistant à analyser l'échantillon de gaz (G) avec l'ensemble capteur (S) comprend d'établir un signal de réponse à partir de l'ensemble capteur (S) et de comparer le signal de réponse avec un signal de référence pour détecter la présence du au moins un composant aromatique (X) dans l'échantillon de gaz (G).

3. Procédé selon la revendication 1, dans lequel l'étape de génération d'un flux ou d'un écoulement de l'échantillon de gaz (G) à travers la zone de test (2) comprend de :
introduire un gaz sous pression au niveau de l'entrée de gaz (I) de la zone de test (2) ; et/ou
connecter la sortie de gaz (O) de la zone de test (2) à une source de sous-pression ou de dépression.

4. Procédé selon l'une quelconque des revendications précédentes, comprenant en outre une étape consistant à :
diluer l'échantillon de gaz (G) en aval du produit (C) avant l'étape d'analyse de l'échantillon de gaz (G) avec l'ensemble capteur (S); et/ou
chauffer l'échantillon de gaz (G) en aval du produit (C) avant l'étape d'analyse de l'échantillon de gaz (G) avec l'ensemble capteur (S).

5. Procédé selon l'une quelconque des revendications précédentes, comprenant en outre une étape de régénération d'un capteur (S1, S2, S3) de l'ensemble capteur (S) après la détection du au moins un composé aromatique (X) dans l'échantillon de gaz (G); dans lequel l'étape de régénération du capteur (S1, S2, S3) comprend de préférence de fournir un gaz propre (CG) à ce capteur.

6. Procédé selon l'une quelconque des revendications précédentes, dans lequel l'ensemble capteur (S) comprend au moins un capteur (S1, S2, S3) choisi dans le groupe constitué par : des capteurs d'oxydes métalliques, des capteurs de thermoconductivité, et des capteurs d'ionisation de flamme.

7. Système (1) pour l'évaluation d'un produit (C), en particulier d'un article à fumer ou d'un élément filtrant d'article à fumer, pour déterminer la qualité du produit et/ou l'intégrité du produit, comprenant :
un moyen de maintien (H) pour maintenir le produit (C) dans une zone de test (2) ayant une entrée de gaz (I) et une sortie de gaz (O);
un moyen pour fournir ou générer un flux d'un échantillon de gaz (G) à travers la zone de test (2) depuis l'entrée (I) vers la sortie (O); et
un ensemble capteur (S) agencé en aval de la zone de test (2) dans laquelle l'échantillon de gaz (G) est dirigé pour détecter un composé aromatique (X) dans l'échantillon de gaz (G),
**caractérisé en ce que**
- la zone de test (2) est située dans un trajet de transport qui est prévu pour acheminer le produit à partir d'un équipement de fabrication vers un équipement de conditionnement; et
- le système (1) comprend en outre une vanne de commande (CV1) pour réguler ou contrôler l'écoulement de l'échantillon de gaz (G) à un débit dans la plage d'environ 0,1 l/min à environ 20 l/min, et dans lequel l'ensemble capteur (S) comprend une pluralité de capteurs (S1, S2, S3) disposés en parallèle, et dans lequel, pour une étape consistant à diriger l'échantillon de gaz (G) vers l'ensemble capteur (S), un trajet d'écoulement du flux de gaz (G) peut être commuté à partir d'un capteur (S1, S2, S3) vers un autre capteur (S1, S2, S3) après que le composé aromatique (X) dans l'échantillon de gaz (G) est détecté.

8. Système (1) selon la revendication 7, dans lequel l'ensemble capteur (S) comprend au moins un capteur (S1, S2, S3) et un dispositif de traitement de signal configuré pour établir un signal de réponse à partir du capteur (51, S2, S3) et pour comparer le signal de réponse avec un signal de référence pour détecter la présence du composé aromatique dans l'échantillon de gaz (G) à partir de la sortie (O) de la zone de test (6).

9. Système (1) selon la revendication 7 ou la revendication 8, dans lequel l'ensemble capteur (S) comprend au moins un capteur (S1, S2, S3) choisi dans le groupe constitué par : des capteurs d'oxydes métalliques, tels que des capteurs d'oxyde d'étain, des capteurs de thermoconductivité, et des capteurs d'ionisation de flamme.

10. Système (1) selon l'une quelconque des revendications 7 à 9, dans lequel le moyen pour générer l'écoulement de l'échantillon de gaz (G) à travers la zone de test (2) comprend un gaz échantillon sous pression connecté pour l'introduction au niveau de l'entrée de gaz (I) de la zone de test (2) et/ou une sous-pression ou une dépression appliquée à la sortie de gaz (O) de la zone de test (2).

11. Système (1) selon l'une quelconque des revendications 7 à 10, dans lequel le moyen de maintien (H) pour maintenir le produit (C) comprend un premier élément de maintien (H1) au niveau de l'entrée (I) de la zone de test (2) et un second élément de maintien (H2) au niveau de la sortie (O) de la zone de test (2), dans lequel les premier et second éléments de maintien (H1, H2) comprennent de préférence un ou plusieurs éléments d'étanchéité (4) pour venir en prise avec le produit et former une étanchéité autour de celui-ci (C).

12. Système (1) selon l'une quelconque des revendications 7 à 11, comprenant en outre :
un moyen pour diluer l'échantillon de gaz (G) en aval de la zone de test (2) et en amont de l'ensemble capteur (S) ; et/ou
au moins un dispositif de chauffage (HE) pour chauffer l'échantillon de gaz (G) en aval de la zone de test (2) et en amont de l'ensemble capteur (S).

13. Système (1) selon l'une quelconque des revendications 7 à 12, comprenant en outre un moyen pour régénérer chaque capteur (S1, S2, S3) de l'ensemble capteur (S), le moyen de régénération comprenant de préférence un moyen pour fournir de l'air, de l'oxygène et/ou de l'azote à chaque capteur (S1, S2, S3).
